# EUROPÄISCHE PATENTSCHRIFT

(11) **EP 2 179 703 B1**
(45) Veröffentlichungstag und Bekanntmachung des Hinweises auf die Patenterteilung: **28.03.2012**
(21) Anmeldenummer: 08167095.2
(22) Anmeldetag: 21.10.2008
(51) Int. Cl.: A61B 19/00, A61B 17/00, A61B 19/08

(54) **Integration von chirurgischem Instrument und Anzeigevorrichtung zur Unterstützung der bildgeführten Chirurgie**
Integration of surgical instrument and display device for supporting image-based surgery
Intégration d'un instrument chirurgical et dispositif d'affichage destiné à l'assistance de la chirurgie assistée par imagerie

(43) Veröffentlichungstag der Anmeldung: 28.04.2010
(60) Teilanmeldung: 12155187.3
(73) Patentinhaber: BrainLAB AG, 85622 Feldkirchen (DE)
(72) Erfinder: Tuma, Gregor, 81677 München (DE); Neubauer, Timo, 85586 Poing (DE)
(74) Vertreter: Schwabe - Sandmair - Marx

(56) Entgegenhaltungen:
- WO-A-98/32388
- WO-A-2004/112610
- FR-A- 2 830 743
- FR-A- 2 852 226
- US-A1- 2008 009 697

## Beschreibung

Die Erfindung betrifft die Integration von chirurgischen Instrumenten und Anzeigevorrichtungen und auch noch einer Recheneinheit zur Unterstützung der bildgeführten Chirurgie. Insbesondere betrifft sie ein chirurgisches Instrument mit einem Griff- oder Halterungsabschnitt und einem funktionellen Abschnitt. Bei speziellen Ausführungsformen der vorliegenden Erfindung ist das chirurgische Instrument ein Zeigeinstrument - ein so genannter Pointer.

Der genannte funktionelle Abschnitt ist derjenige Abschnitt, mit dem das Instrument gemäß seiner Aufgabe ausgestattet ist, also beispielsweise eine Zeigerspitze bei einem Pointerinstrument, eine Schneide bei einem Skalpell, ein Befestigungsabschnitt (Der Befestigungsabschnitt ist geeignet, um das Instrument woanders, insbesondere an anderen medizintechnischen Instrumenten anbringen zu können, z. B. an einem Schneidblock, einem Implantat, einer Fräse, einer Säge, einem Bohrer, einem Meißel, einem Schraubendreher usw.) oder ein zangenartiger Abschnitt bei einer Pinzette. Sie kann auch eine Kalibrierungsaufnahme sein oder umfassen, die ein Gegenstück für ein zum Instrument auszurichtendes Objekt aufweist, z.B. zum schnellen Registrieren eines Bilddatensatzes mit gescanntem Gegenstück. Diese funktionellen Abschnitte werden im täglichen Sprachgebrauch auch oft als "Spitze" des Instruments bezeichnet, und der Begriff "Spitze" wird in diesem Sinne hierin auch verwendet. Er kann also auch funktionelle Abschnitte von Instrumenten bezeichnen, die nicht körperlich als Spitze oder spitz ausgestaltet sind.

Die oben genannten Pointer werden im Rahmen der navigationsgestützten bzw. bildunterstützten Chirurgie in vielen Fällen dazu verwendet, eine Patientenregistrierung durchzuführen. Dabei werden verschiedene Punkte bzw. Landmarken am Patienten angefahren, und zwar mit einem räumlich getrackten Pointer, um die Raumlage dieser Punkte bzw. Landmarken im Navigations-Koordinatensystem eines Behandlungsraumes festzustellen. Diese Punkte oder Landmarken werden dann entsprechenden Punkten oder Landmarken zugeordnet, die in einem beispielsweise vor der Behandlung akquirierten Bilddatensatz (CT, MRI, etc.) vorhanden und im Navigationssystem abgespeichert sind. Auf dieser Basis kann dann die navigationsunterstützte bzw. bildunterstützte Behandlung ausgeführt werden. Räumlich verfolgt werden die Instrumente, also auch der Pointer, in vielen Fällen durch optische Trackingsysteme, wobei an den Instrumenten angebrachte Markierungen (Trackingmarker) von Kameras verfolgt werden.

Diese Abfolge von Registrierungsschritten ist mit getrackten Pointerinstrumenten gemäß dem Stand der Technik und herkömmlichen Navigationssystemen eher mühsam zu bewältigen, weil der Behandelnde andauernd den Blickwinkel ändern muss. Um nachzusehen, welcher Schritt als nächstes in der Registrierungsprozedur folgt, muss auf den Navigationsmonitor geblickt werden, während parallel dazu die Landmarke am Patienten gesucht wird. Um sicher zu gehen, dass die Landmarke akquiriert worden ist, muss der Chirurg wiederum auf den Monitor blicken oder ein Bestätigungssignal abwarten. Wenn mehrere Punkte oder Punktwolken (bei einer Oberflächenakquirierung) abgetastet werden müssen, wird die Situation noch schwieriger, weil die richtige Pointerstellung mit Hilfe des Monitors - gewöhnlich weit entfernt vom Arbeitsumfeld - immer wieder verifiziert werden muss.

Es ist eine Aufgabe der vorliegenden Erfindung, die Handhabung eines chirurgischen Instruments im Navigationsumfeld zu erleichtern, insbesondere auch den Umgang mit dem Instrument für den Chirurgen im Navigationsumfeld einfacher zu gestalten. Dies gilt speziell auch für Registrierungsaufgaben.

Diese Aufgabe wird erfindungsgemäß durch ein chirurgisches Instrument gemäß dem Anspruch 1 gelöst. Die Unteransprüche definieren bevorzugte Ausführungsformen der Erfindung.

An dem chirurgischen Instrument gemäß der vorliegenden Erfindung, das einen Griff-oder Halterungsabschnitt sowie eine Spitze aufweist, ist eine Anzeigevorrichtung vorgesehen, welche Anzeigen umfasst oder ermöglicht, die der Unterstützung der bildgeführten bzw. navigationsgestützten Chirurgie dienen. Mit anderen Worten zeigt also das Instrument selbst Informationen für den Chirurgen an, die er während des Behandlungsablaufes benötigt, so dass der Chirurg nicht den Blick vom Instrument bzw. vom Patienten nehmen muss, um navigationsgestützt arbeiten zu können. Ein Registrierungsverfahren wird beispielsweise dabei derart unterstützt, dass während der Landmarken-Akquirierungssequenz die Arbeit sehr viel intuitiver vonstatten gehen kann. Pointer oder Zeigegeräte, die erfindungsgemäß ausgestaltet sind, können nicht nur Landmarken oder Punkte positionell akquirieren, sondern ebenfalls Informationen über den Ort der Landmarke selbst zur Verfügung stellen. Der Chirurg kann seine Aufmerksamkeit auf den Behandlungsort, also das Einschnittfeld konzentrieren; er wird nicht durch Blicke zum Monitor abgelenkt.

Die Anzeigevorrichtung kann im Rahmen der vorliegenden Erfindung unterschiedlichste Gestaltungen aufweisen. Einerseits kann sie eine oder mehrere hervorhebbare Kennzeichnungen umfassen, insbesondere beleuchtbare oder hinterleuchtbare Symbole oder Schriftzeichen. Erfindungsgemäß weist sie eine Bildanzeige, insbesondere eine Bildschirmanzeige auf, welche in unterschiedlichster Form ausgestaltet sein kann. Hier sind hoch auflösende Farbmonitore aber auch Strom sparende LCD-Bildanzeigen möglich. Ganz allgemein ist zu sagen, dass es im Rahmen der Erfindung wichtig ist, mit der Anzeige Informationen für den Arzt bereitzustellen, der ansonsten auf den Monitor blicken müsste. Dies kann auch Leuchtanzeigen umfassen, die punkt- oder streifenartig ausgebildet sind und beispielsweise Lastfälle mit Rot-Gelb-Grün-Anzeigen vermitteln.

Die Position des chirurgischen Instruments wird bei einer erfindungsgemäßen Ausführungsform mittels eines medizintechnischen Trackingsystems bestimmt und verfolgt bzw. getrackt wobei die Positionsdaten im Rahmen einer medizintechnischen Navigation mittels eines medizintechnischen Navigationssystems verarbeitet werden.

Erfindungsgemäß ist eine Datenverarbeitung im Instrument integriert und der Anzeigevorrichtung zugeordnet, wobei mittels der Datenverarbeitung die Tätigkeit des Instruments verfolgt und identifiziert wird. Mit anderen Worten erfolget im Instrument die Auswertungen der Navigations- und Trackingdaten die wiederum über die Anzeigevorrichtung dem Chirurgen bereitgestellt werden.

Die Anzeigevorrichtung ist an dem Instrument über einen Adapter anbringbar.

Instrumente gemäß der vorliegenden Erfindung können mit Trackingmarkierungen versehen werden, insbesondere können diese Trackingmarkierungen auch im Bereich oder an der Anzeigevorrichtung angebracht werden. Dies schließt den Fall ein, wo Reflektionsmarker oder Infrarot-LEDs starr an dem Instrument befestigt werden, beispielsweise zwei oder drei Tackingmarker in festgelegter geometrischer (charakteristischer) Anordnung zueinander.

Die Bereitstellung solcher Markeranordnungen bzw. Referenzanordnungen ist aber aufwendig und erfordert Kalibrierungsschritte. Ein Tracking lediglich anhand einer Objektform ist wiederum nicht robust genug gegen Störungen in den Bildern, im Hintergrund oder im Kontrast, etc.

Für Abhilfe sorgt die vorliegende Erfindung hier mit einer Ausführungsform des chirurgischen Instruments, bei welcher die Anzeige selbst Tracking-Markierungen anzeigt oder selbst Tracking-Markierungen oder eine Tracking-Markierung verkörpert. Mit anderen Worten wird gemäß dieser Ausführungsform der Erfindung ein chirurgisches Instrument oder eine Trackingreferenz (z.B. Knochen-Tracker) bereitgestellt, das automatisch getrackt werden kann oder dessen Anzeige-Inhalt getrackt wird. Dadurch entfällt die Notwendigkeit, Trackingmarkierungen anzubringen, aber das Tracking wird trotzdem noch robust genug sein, um das Instrument mit der Anzeigevorrichtung durchgängig und verlässlich positionell zu verfolgen. Die Anzeige kann nämlich ohne Weiteres hell genug gemacht werden, um ihre Detektion durch das Kamerasystem sicherzustellen. Sie kann auch durch Farb-/Form- oder Mustergebung eindeutig genug gemacht werden. Wenn beispielsweise die Abmessungen des Bildschirmrechtecks im Navigationssystem bekannt sind, kann die Ausrichtung und Position aus der Blickwinkelverzerrung in Relation zu einem interessierenden Punkt am Instrument oder zur anatomischen Struktur bestimmt werden. Weiterhin kann die Anzeigeinformation unter Umständen direkt gematcht werden, da diese eindeutig beschreibbar ist. Außerdem wird die Einheit, bei der die Trackingmarkierungen auf der Anzeigevorrichtung angezeigt werden, dazu in der Lage sein, das Tracking-Muster automatisch oder manuell zu verändern, um eine Anpassung an bestimmte Tracking-Situationen vorzunehmen oder unterschiedliche Instrumente unterschiedlichen speziellen Tracking-Mustern zuzuordnen, die dann parallel getrackt werden können.

Bei einer erfindungsgemäßen Ausführungsform umfasst die Anzeigevorrichtung die Navigations-Bildanzeige. Mit dieser erfindungsgemäßen Ausführungsform wird es für den Arzt möglich, seine gesamte Navigation bildunterstützt vorzunehmen, ohne einmal den Blick vom Arbeitsfeld, in dem sich das Instrument befindet, nehmen zu müssen.

Erfindungsgemäß ist auch die Datenverarbeitung des Navigationssystems an dem Instrument anbringbar, speziell über einen Adapter. Der Arzt hat dann ein tragbares Navigationssystem mit Anzeige und Datenverarbeitung an seinem Instrument; die Bereitstellung der übrigen Navigationskomponenten beschränket sich auf die Bereitstellung des Kamera-Trackingsystems.

Die integrierte Datenverarbeitung kann im Instrument die Auswertung einer Inertialsensorik übernehmen, insbesondere in Verbindung mit redundanter Trackinginformation (z.B. Kalman-Filterung), um eine Verbesserung der 3D-Lokalisation zu erwirken.

Eine Ausführungsform eines erfindungsgemäßen Instruments ist dadurch gekennzeichnet, dass die Datenverarbeitung und die Anzeigevorrichtung als integriertes, speziell tragbares Navigationssystem bereitgestellt sind. Dieses integrierte System, insbesondere das integrierte Navigationssystem kann am Griff- oder am Halterungsabschnitt des Instruments abnehmbar über den Adapter anbringbar sein. Im Falle eines direkten Trackings der Anzeigevorrichtung ist beispielsweise der Tracker auf der Anzeige und der Adapter ohne Tracker, im Falle eines Trackings über z.B. Markerkugeln sind diese entweder an der Anzeigevorrichtung oder am Adapter. Die Adaption erfolgt vorzugsweise in bekannter räumlichen Zuordnung zueinander von Instrument und Adapter und lässt sich reproduzierbar lösen und wiederverbinden.

Das Instrument kann als Spitze eine funktionelle Instrumentenspitze haben (z.B. Pointerinstrument); es kann aber auch als Spitze ein Befestigungsabschnitt zum Fixieren des Instruments bereitgestellt werden, so dass das Instrument dann selbst ein Tracker (eine Trackingmarkierung/-referenz) wird, der beispielsweise an einem Knochen befestigt werden kann.

Offenbart wird few ein Verfahren zum Navigieren eines chirurgischen Instruments, insbesondere eines Instruments, wie es oben in verschiedenen Ausführungsformen beschrieben worden ist. Die Position des Instruments wird mittels eines medizintechnischen Trackingsystems bestimmt und verfolgt bzw. getrackt, und die Positionsdaten werden im Rahmen einer medizintechnischen Navigation mittels eines medizintechnischen Navigationssystems verarbeitet, wobei Anzeigen zur Navigationsunterstützung bzw. zur Unterstützung einer bildgeführten Chirurgie am Instrument oder einem ihm positionell zugeordneten oder an ihm befestigten Element erfolgen.

Wenn mit dem Instrument, das als Positionszeiger bzw. Pointer dient, Landmarken oder Oberflächen akquiriert und registriert werden, kann die Anzeigevorrichtung die Auswahl der Landmarken bzw. Oberflächen vorgeben oder unterstützen. Dies gilt grundsätzlich auch für die Abfolge der Akquirierung.

Es ist grundsätzlich möglich, die Anzeigevorrichtung durch eine Datenverarbeitung anzusteuern, welche Bewegungen oder Punktakquirierungen mit Hilfe des Navigationssystems oder durch Sensoren am Instrument feststellt. Es ist auch möglich, Bewegungen oder Punktakquirierungen mit Hilfe einer definierten Bewegung der Tracker zueinander festzustellen.

Die bildliche Navigationsunterstützung kann über eine Anzeigevorrichtung ausgegeben werden, die als Bildschirm ausgebildet ist, und die Anzeigevorrichtung kann - wie oben schon beschrieben - wechselnde Trackingmarkierungen anzeigen. Ferner ist es im Rahmen der Erfindung möglich, dass die bildliche Navigationsunterstützung über die Anzeigevorrichtung gleichzeitig die Trackingmarkierungen darstellt.

Das erfindungsgemäße Instrument kann so gestaltet werden, dass die Anzeigevorrichtung oder ein im Instrument integriertes Navigationssystem, welches die Anzeigevorrichtung umfassen kann, als separates am Instrument anbringbares Element bereitgestellt ist, das eine sterile Umhüllung, insbesondere ein steriles Drape oder eine sterile bzw. sterilisierbare Schale, aufweist. Ferner ist es denkbar, dass der Griff- oder Halterungsabschnitt und/oder der funktionelle Abschnitt und insbesondere auch daran angebrachte Bestandteile des Instruments als sterile Wegwerfartikel, insbesondere als steril verpackte Wegwerfartikel ausgebildet sind, speziell aus einem Kunststoffmaterial.

Die Erfindung wird im Weiteren anhand der beiliegenden Zeichnungen und unter Bezugnahme auf verschiedene Ausführungsformen näher erläutert. In den beiliegenden Zeichnungen zeigen:
- Figuren 1 bis 3: Pointerinstrumente mit unterschiedlichen Anzeigevorrichtungen;
- Figuren 4 und 5: erfindungsgemäße Pointer mit einem Navigationsbildschirm, der auch als Trackingreferenz dient;
- Figur 6: eine Ausführungsform der Erfindung als Knochen-Tracker,
- Figur 7: ein erfindungsgemäßes Instrument als tragbares Navigationssystem mit einer Wegwerf- oder Einmal-Halterung (sterile Lösung); und
- Figur 8: eine weitere Ausführungsform eines steril verpackten, tragbaren Navigationssystems.

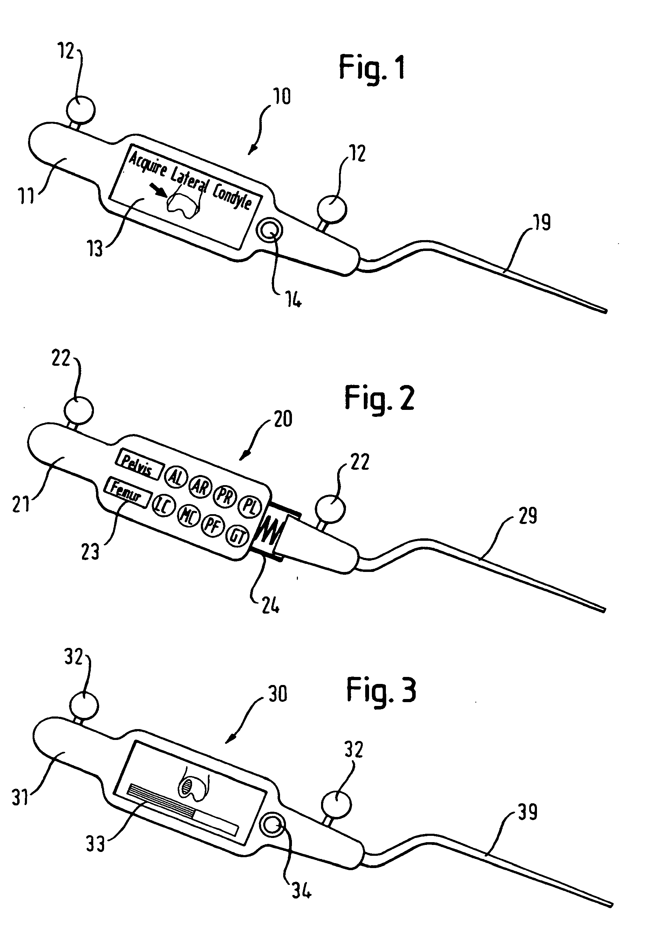

In den Figuren 1 bis 7 sind die chirurgischen Instrumente ganz allgemein jeweils mit dem Bezugszeichen 10, 20, 30, 40, 50 60 und 70 versehen worden. Die Griffe der Instrumente 10, 20 und 30 tragen die Bezugszeichen 11, 21 und 31, während die Halterungsabschnitte der Instrumente 40, 50, 60 und 70 jeweils die Bezugszeichen 41, 51, 61 und 71 haben. Letztere Halterungsabschnitte 41, 51, 61 und 71 sind als Adapterhalterungen für Anzeige- und/oder Datenverarbeitungseinheiten, insbesondere tragbare Navigationssysteme 45, 55, 65, 75 vorgesehen, sie können aber auch feste Halterungen sein. Die Anzeigen tragen durch die Figuren jeweils die Bezugszeichen 13, 23, 33, 43, 53, 63 und 73 und Marker bzw. Trackingmarkierungen sind mit 12, 22, 32, 42, 52, 72 und 82 gekennzeichnet. In den Figuren 1 und 3 deuten die Bezugszeichen 14 und 34 auf Knöpfe am Instrument, welche die Anzeige beeinflussen oder Eingaben am Instrument ermöglichen, die auch an ein externes Navigationssystem weitergeleitet werden können.

In der Figur 2 deutet das Bezugszeichen 24 auf eine federnde Halterung der Instrumentenspitze 29. Die Feder stellt die Möglichkeit einer automatischen Punktaufnahme dar, die dem Trackingsystem durch definierte Verschiebung von Markerkugeln zueinander oder durch Kraftsensorik bekannt gemacht wird.

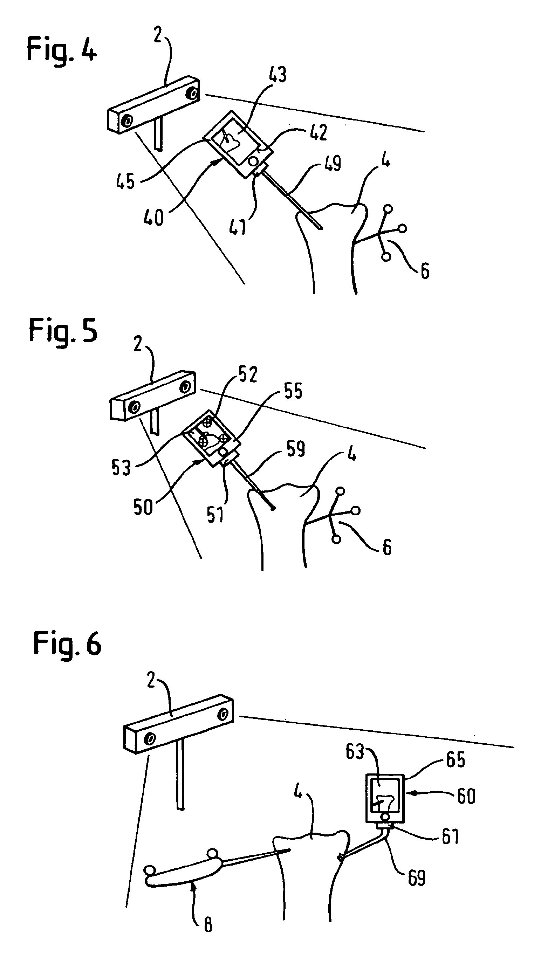

Die Instrumentenspitzen 19; 29, 39, 49 und 59 sind in den Figuren 1 bis 5 als Pointer- bzw. Zeigerspitzen ausgestaltet, während die Figur 6 eine Instrumentenspitze 69 zeigt, die an ihrem distalen Ende eine Befestigungseinrichtung aufweist. In den Figuren 1 bis 3 deuten die Bezugszeichen 12, 22 und 32 auf Kugel-Referenzmarker, das Bezugszeichen 42 in Figur 4 deutet auf den äußeren Umriss der Bildanzeige 43, und das Bezugszeichen 52 in Figur 5 deutet auf Referenzmarker, die auf der Bildanzeige 53 erzeugt werden. In den Figuren 4, 5 und 6 ist noch eine Trackingkamera 2 mit zwei Einzelkameras zu sehen, jeweils ein Knochen 4, in den Figuren 4 und 5 eine Referenzanordnung 6 mit Referenzmarkern für den Knochen (Knochen-Tracker), und die Figur 6 zeigt noch ein weiteres Pointerinstrument 8.

Zunächst soll im Weiteren auf die Figuren 1 bis 3 Bezug genommen werden. Hier ist jeweils eine Anzeige, 13, 23, 33 vorgesehen, die dem Arzt Instruktionen vermittelt, wo ein Punkt auf der Patientenanatomie in einem Registrierungsvorgang zu akquirieren ist. Dies könnte entweder durch graphische Darstellungen oder Text geschehen (Figur 1) oder in Zusammenwirkung mit einer Anzeige, die darstellt, ob die Landmarke noch akquiriert werden muss oder schon akquiriert worden ist. Letzteres gilt beispielsweise für die hinterleuchteten Knopfanzeigen der Figur 2, wo z.B. ein rot hinterleuchteter Knopf anzeigt, dass eine gewisse Landmarke noch akquiriert werden muss und ein grün hinterleuchteter Knopf bedeutet, dass die Landmarkenakquirierung schon stattgefunden hat. Die Anzeige kann, wie in Figur 3, auch darüber Instruktionen geben, wie viele Punkte schon akquiriert worden sind oder welcher Anteil einer Gesamt-Punktakquirierung bisher schon erzielt worden ist (Balkenanzeige). Dies sagt dem Verwender, wo und wie viele Punkte bzw Punktewolken noch mit der Pointerspitze abgefahren werden müssen, um beispielsweise eine Knochenoberfläche zu registrieren, d. h. insbesondere für ein Morphing oder Surface-Matching mit einem Bilddatensatz.

Die in den Figuren 1 bis 3 dargestellten Pointerinstrumente verwenden reflektive Trackingmarker 12, 22, 32, die von einem Trackingsystem (z.B. Infrarotkamerasystem) erfasst werden können. Es ist möglich, andere Trackingtechnologien zu verwenden, beispielsweise das Tracking aktiver LED-Marker, elektromagnetisches Tracking oder akustisches Tracking.

Die Pointerinstrumente gemäß der vorliegenden Erfindung weise nicht nur Anzeigen auf, sondern sind auch mit einer Datenverarbeitungseinheit ausgestattet sein, die softwaregesteuert ist und die Akquirierung eines Punktes oder einer Punktewolke autonom bzw. mit Hilfe des Navigationssystems detektieren kann. Beispielsweise könnten Trägheits-Beschleunigungssensoren die momentane algorithmische Integration einer Schwenkbewegung des Pointers detektieren, um den Punkt zu akquirieren, und als Konsequenz die Anzeige auf dem Display von einem Registrierungsbildschirm zum nächsten weiterschalten. In gleicher Weise könnten die diskreten Anzeigen der Ausführungsform nach Figur 2 angesteuert werden. In dem Fall ist eine weitere Kommunikation mit dem Navigationssystem nicht mehr notwendig, sie kann aber als Option bereitstehen und durch die Knöpfe 14, 34 realisiert werden. Eine weitere Möglichkeit der Realisierung wäre die Verkürzung des Abstandes der beiden Markerkugeln 22 in der Ausführungsform nach Figur 2, wenn mit der Spitze 29 ein Punkt angezeigt wird und die Spitze dabei zum Griff hin einfedert (federnde Lagerung 24).

In den Figuren 4 bis 6 ist jeweils dargestellt, wie ein Navigationssystem bereitgestellt wird, das aus einer Datenverarbeitungs- und Anzeigeeinheit 45, 55, 65 und einer extern bereitgestellten Trackingkamera 2 besteht. Die Einheiten 45, 55 und 65 können dabei diskrete, separate und vollständig integrierte Navigationseinheiten bzw. Anzeigeeinheiten sein. Sie sind über Adapterhalterungen 41, 51 und 61 am Instrument befestigt. In den Fällen der Figuren 4 und 5 hat das Instrument eine Pointerspitze 49, 59; im Fall der Figur 6 haltert die Spitze 69 das Instrument am Knochen 4. Durch die Navigationseinheiten 45, 55 und 65 werden Navigationsinformationen oder Verwenderanleitungen dargestellt, und dies könnte entweder durch graphische Darstellungen oder Textinformation geschehen. Damit die Instrumente im Raum mit dem optischen Trackingsystem 2 positionell verfolgt werden können, wird bei der Ausführungsform nach Figur 4 der Umriss 42 des Bildschirms bzw. der Anzeige 43 und seine dreidimensionale Ausrichtung mit dem Kamerasystem 2 erfasst.

Als Alternative wird bei der Ausführungsform gemäß Figur 5 die Darstellung der Trackingmarker 52 direkt auf der Anzeige 53 erfolgen; es können also spezielle Muster erzeugt werden, die "virtuelle Marker" erzeugen. Ein Muster kann die Anzeige selbst sein, da die Anzeige zumindest in Teilaspekten durch die Programmierung der grafischen Benutzeroberfläche festgelegt ist. Die Erhöhung der Robustheit durch bekannte grafische Darstellungen kann auch dazu führen, dass normale Kameras verwendet werden können (statt Infrarot). Diese erfindungsgemäßen Lösungen gestatten die Vereinigung der Informations-Anzeige mit den Tracker-Einheiten innerhalb der Sichtlinie zum Trackingsystem 2. Eine zusätzliche Anbringung von Trackingmarkern ist nicht mehr notwendig.

Während die beiden Instrumente 40, 50 der Figuren 4 und 5 als Pointerinstrumente zur Landmarken-Registrierung dienen können, bei der die Patientenanatomie noch separat getrackt wird (Knochen 4 mit Trackingreferenz 6), kann gemäß der Ausführungsform nach Figur 6 das Trackingsystem selbst an dem Instrument und mit dem Instrument am Knochen 4 befestigt werden. Dadurch ist es möglich, den Knochen 4 über das Instrument 60 selbst zu tracken, und zwar über das Anzeige-Rechteck (siehe Figur 4) oder über eingeblendete Marker (siehe Figur 5). Das Instrument 60 mit dem Navigations- und Anzeigesystem 65 dient also sowohl als Trackingreferenz als auch als Anzeige für den Chirurgen, und gleichzeitig kann ein weiteres Pointerinstrument 8 getrackt werden.

In allen Fällen kann der behandelnde Chirurg alle Navigationsinformationen erhalten, ohne andauernd vom Arbeitsfeld weg und auf einen separaten Navigationsmonitor blicken zu müssen.

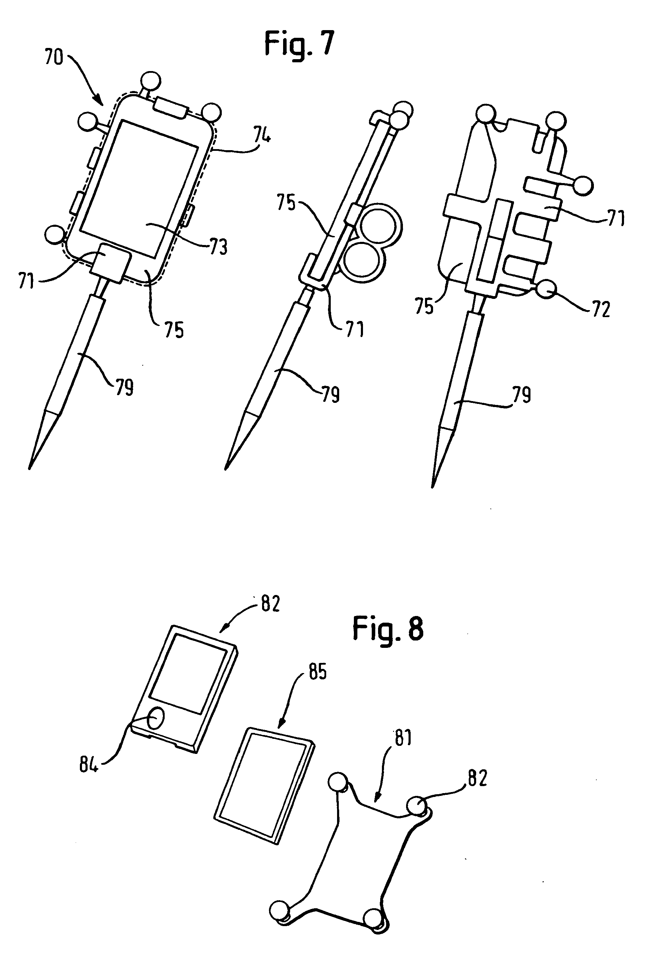

Die Figuren 7 und 8 verdeutlichen erfindungsgemäße "Sterillösungen", d.h. Ausführungsformen, bei denen ein erfindungsgemäßes Instrument mit einer Anzeige oder einem daran angebrachten tragbaren Navigationssystem in einfacher und unkomplizierter Weise steril eingesetzt werden kann, und zwar mit Hilfe von Einweg-oder Wegwerf-Komponenten für Griff- oder Halterungsabschnitte bzw. funktionelle Abschnitte (Spitze).

Mit derartigen erfindungsgemäßen Ausführungsformen ist es möglich, eine Kombination eines Navigationssystems 75 (mit Anzeige 73), 85 und eines chirurgischen Instruments 70 in einer sterilen Umgebung zu verwenden. Dabei werden bei der Ausführungsform nach Figur 7 die Halteeinrichtung 71 bzw. die Spitze 79 (einzeln oder beide) als Einweg- bzw. Wegwerfartikel (einmal verwendbar) ausgebildet, die auch schon Navigations-Referenzmarker 72 tragen können. Man würde diesen Teil des Instruments einfach schon steril verpackt in der Operationsumgebung zur Verfügung stellen und nach einmaliger Verwendung entsorgen können. Er kann im Wesentlichen oder vollständig aus Kunststoff bestehen. Der Halterungsabschnitt 71 bildet einen Rahmen, in den das tragbare Navigationssystem 75 eingesetzt werden kann; er umfasst ferner Referenzmarker 72 und an ihm können alle möglichen Instrumentenspitzen angebracht sein (hier ist eine Pointerspitze 79 dargestellt). Es kann sich hierbei aber auch um eine am Halterungsabschnitt 71 angebrachte L-förmige Vorrichtung (zur Registrierung der distalen Gelenklinie und der posterioren Gelenklinie) oder um einen Schneidblock handeln.

Bei der Ausführungsform nach Figur 7 ist das tragbare Navigationssystem 75 mit der Anzeige 73 vor dem Einsetzen in die Halterung 71 in ein steriles Drape (Folienumhüllung) eingehüllt worden; dieses Drape ist gestrichelt angedeutet und hat das Bezugszeichen 74 erhalten. Auf diese Weise wird also das Navigationssystem 75 (mit Anzeige 73) ebenso steril bereitgestellt und kann unmittelbar zusammen mit den restlichen sterilen Elementen des Instruments verwendet werden. Das gesamte Instrument ist so ausgestaltet, dass der Verwender leicht mit dem Navigationssystem oder der Anzeige interagieren kann (z.B. berührungsempfindlicher Bildschirm 73), während es in der Hand gehalten und als chirurgisches Instrument verwendet wird.

Wie in Figur 8 dargestellt ist, kann die Halteeinrichtung auch eine Halteplatte 81 mit Navigationsmarkern 82 aufweisen, auf der ein Navigationssystem (mit Anzeige 85) aufgebracht wird, die mit einer sterilen, starre Umhüllung (beispielsweise Abdeckung aus durchsichtigem Kunststoffmaterial) abgedeckt wird. Die Bedienung des Navigationssystems kann dann beispielsweise über dünnere Umhüllungsstellen 84 (auch im Bereich des Bildschirms möglich) erfolgen, die ein Durchtasten ermöglichen.

Insgesamt können erfindungsgemäß alle Teile des so erzeugten Instruments gemäß Figur 7 oder 8 als Einweg-Teile bereitgestellt werden, einschließlich Halterungsabschnitt, Instrumentenspitze und Referenzmarkierungen, so dass das Instrument keinen Sterilisierungsaufwand mehr erfordert. Dies gilt speziell, wenn auch die sterile Abdeckung 83, 74 als Einweg- bzw. Wegwerfartikel bereitgestellt wird.

## Patentansprüche

1. Chirurgisches Instrument (10, 20, ..., 60) mit einem Griff- oder Halterungsabschnitt (11, 21, ..., 61) und einem funktionellen Abschnitt bzw. einer Spitze (19, 29, ..., 69), wobei am Instrument eine Anzeigevorrichtung (13, 23, ..., 63) mit einer Bildanzeige vorgesehen ist, welche Anzeigen umfasst oder ermöglicht, die der Unterstützung der bildgeführten bzw. navigationsgestützten Chirurgie dienen, wobei die die Positionsdaten des Instruments im Rahmen einer medizintechnischen Navigation verarbeitende Datenverarbeitung des Navigationssystems und die Anzeigevorrichtung als integriertes, speziell tragbares Navigationssystem über einen Adapter an dem Instrument anbringbar sind, wobei die Position des Instruments zur 3D-Lokalisation mittels einer extern bereitgestellten Trackingkamera (2) bestimmt und verfolgt bzw. getracht wird, **dadurch gekennzeichnet, dass** mittels der Datenverarbeitung die Verarbeitung der Navigations - und Trackingdaten der Kamera erfolgt.

2. Instrument nach Anspruch 1, **dadurch gekennzeichnet, dass** die Anzeigevorrichtung (23) eine oder mehrere hervorhebbare Kennzeichnungen umfasst, insbesondere beleuchtbare oder hinterleuchtbare Symbole oder Schriftzeichen.

3. Instrument nach einem der Ansprüche 1 oder 2, **dadurch gekennzeichnet, dass** eine eingebaute Sensorik, speziell Inertialsensorik, Trackinginformation liefert.

4. Instrument nach Anspruch 3, **dadurch gekennzeichnet, dass** die integrierte Datenverarbeitung im Instrument die Auswertung einer Inertialsensorik in Verbindung mit der redundanten Trackinginformation (z.B. Kalman-Filterung) übernimmt, um eine Verbesserung der 3D-Lokalisation zu erwirken.

5. Instrument nach einem der Ansprüche 1 bis 4, **dadurch gekennzeichnet, dass** die Adaption in bekannter räumlichen Zuordnung zueinander von Instrument und Adapter erfolgt und reproduzierbar lösbar und wiederverbindbar ist.

6. Instrument nach einem der Ansprüche 1 oder 3 bis 5, **dadurch gekennzeichnet, dass** die Anzeige (13, 33, ..., 63) Tracking-Markierungen für das Trackingsystem aufweist, anzeigt oder selbst eine Tracking-Markierung verkörpert.

7. Instrument nach einem der Ansprüche 1 bis 6, **dadurch gekennzeichnet, dass** die Anzeigevorrichtung (43, 53, 63) die Navigations-Bildanzeige umfasst.

8. Instrument nach Anspruch 1, **dadurch gekennzeichnet, dass** das integrierte System, insbesondere das integrierte Navigationssystem am Griff-oder am Halterungsabschnitt abnehmbar über einen Adapter anbringbar ist.

9. Instrument nach einem der Ansprüche 1 bis 8, **dadurch gekennzeichnet, dass** die Spitze eine funktionelle Instrumentenspitze ist, einen Befestigungsabschnitt zum Fixieren des Instruments aufweist oder eine Kalibrierungsaufnahme ist, die ein Gegenstück für ein zum Instrument auszurichtendes Objekt aufweist.

10. Instrument nach einem der Ansprüche 1 bis 9, **dadurch gekennzeichnet, dass** das integrierte Navigationssystem, welches die Anzeigevorrichtung umfasst, als separates am Instrument anbringbares Element bereitgestellt ist, das eine sterile Umhüllung, insbesondere ein steriles Drape oder eine sterile bzw. sterilisierbare Schale, aufweist.

11. Instrument nach einem der Ansprüche 1 bis 10, **dadurch gekennzeichnet, dass** der Griff- oder Halterungsabschnitt und/oder der funktionelle Abschnitt und insbesondere auch daran angebrachte Bestandteile des Instruments als sterile Wegwerfartikel, insbesondere als steril verpackte Wegwerfartikel ausgebildet sind, speziell aus einem Kunststoffmaterial.

## Claims

1. A surgical instrument (10, 20, ..., 60) comprising a handle portion or mounting portion (11, 21, ..., 61) and a functional portion and/or tip (19, 29, ..., 69), wherein a display device (13, 23, ..., 63) comprising an image display is provided on the instrument and includes or enables displays which serve to assist in image-guided and/or navigation-assisted surgery, wherein a data processor of the navigation system which processes the position data of the instrument within the framework of medical navigation and the display device can be attached, as an integrated, specifically portable navigation system, to the instrument via an adaptor, wherein for 3D localisation, the position of the instrument is determined and tracked by means of an externally provided tracking camera (2), **characterised in that** the navigation and tracking data of the camera are processed by means of the data processor.

2. The instrument according to claim 1, **characterised in that** the display device (23) includes one or more indicators which can be highlighted, in particular symbols or characters which can be illuminated or back-lit.

3. The instrument according to any one of claims 1 or 2, **characterised in that** an integrated sensor system, specifically an inertial sensor system, provides tracking information.

4. The instrument according to claim 3, **characterised in that** the integrated data processor in the instrument assumes the function of evaluating an inertial sensor system in connection with the redundant tracking information (for example, Kalman filtering), in order to improve 3D localisation.

5. The instrument according to any one of claims 1 to 4, **characterised in that** adapting is performed in a known spatial assignment of the instrument and the adaptor with respect to each other and can be reproducibly released and reconnected.

6. The instrument according to any one of claims 1 or 3 to 5, **characterised in that** the display (13, 33, ..., 63) comprises or displays tracking markings for the tracking system, or itself represents a tracking marking.

7. The instrument according to any one of claims 1 to 6, **characterised in that** the display device (43, 53, 63) includes the navigation image display.

8. The instrument according to claim 1, **characterised in that** the integrated system, in particular the integrated navigation system, can be removably attached to the handle portion or mounting portion via an adaptor.

9. The instrument according to any one of claims 1 to 8, **characterised in that** the tip is a functional instrument tip, comprises a fastening portion for fixing the instrument, or is a calibration receptacle which comprises a counter piece for an object to be aligned to the instrument.

10. The instrument according to any one of claims 1 to 9, **characterised in that** the integrated navigation system which includes the display device is provided as a separate element which can be attached to the instrument and comprises a sterile covering, in particular a sterile drape or a shell which is sterile and/or can be sterilised.

11. The instrument according to any one of claims 1 to 10, **characterised in that** the handle portion or mounting portion and/or the functional portion and in particular also components of the instrument which are attached to them are formed as sterile disposable items, in particular as sterilely packaged disposable items, specifically made of a plastic material.

## Revendications

1. Instrument chirurgical (10, 20, ..., 60) comportant une partie de poignée ou de fixation (11, 21, ..., 61) et une partie fonctionnelle ou un embout (19, 29, ..., 69), dans lequel un dispositif d'affichage (13, 23, ..., 63) muni d'un affichage d'images est prévu sur l'instrument, lequel dispositif d'affichage inclut ou permet des affichages qui servent d'aide à la chirurgie guidée par image ou assistée par navigation, dans lequel le système de traitement de données du système de navigation qui traite les données de position de l'instrument dans le cadre d'une navigation médicale et le dispositif d'affichage peuvent être fixés sur l'instrument par l'intermédiaire d'un adaptateur, sous la forme d'un système de navigation intégré, en particulier portatif, dans lequel la position de l'instrument pour la localisation 3D est déterminée et suivie ou atteinte au moyen d'une caméra de suivi (2) agencée à l'extérieur, **caractérisé en ce que** le traitement des données de navigation et de suivi de la caméra est effectué au moyen du système de traitement de données.

2. Instrument selon la revendication 1, **caractérisé en ce que** le dispositif d'affichage (23) inclut un ou plusieurs indicateurs pouvant être mis en valeur, en particulier des symboles ou des caractères pouvant être éclairés ou rétro-éclairés.

3. Instrument selon l'une quelconque des revendications 1 ou 2, **caractérisé en ce qu'**un système de capteur intégré, en particulier un système de capteur inertiel, fournit des informations de suivi.

4. Instrument selon la revendication 3, **caractérisé en ce que** le système de traitement de données intégré dans l'instrument assure l'analyse d'un système de capteur inertiel en liaison avec les informations de suivi redondantes (filtrage de Kalman, par exemple) afin d'améliorer la localisation 3D.

5. Instrument selon l'une quelconque des revendications 1 à 4, **caractérisé en ce que** l'adaptation est réalisée dans une affectation spatiale connue de l'instrument et de l'adaptateur l'un par rapport à l'autre et peut être libérée et reconnectée de manière reproductible.

6. Instrument selon l'une quelconque des revendications 1 ou 3 à 5, **caractérisé en ce que** l'affichage (13, 33, ..., 63) comporte ou affiche des marquages de suivi pour le système de suivi, ou représente lui-même un marquage de suivi.

7. Instrument selon l'une quelconque des revendications 1 à 6, **caractérisé en ce que** le dispositif d'affichage (43, 53, 63) inclut l'affichage d'images de navigation.

8. Instrument selon la revendication 1, **caractérisé en ce que** le système intégré, en particulier le système de navigation intégré, peut être fixé sur la partie de poignée ou de fixation de manière amovible, par l'intermédiaire d'un adaptateur.

9. Instrument selon l'une quelconque des revendications 1 à 8, **caractérisé en ce que** l'embout est un embout d'instrument fonctionnel, comporte une partie de fixation pour la fixation de l'instrument ou est un logement de calibrage qui inclut une pièce complémentaire pour un objet à orienter par rapport à l'instrument.

10. Instrument selon l'une quelconque des revendications 1 à 9, **caractérisé en ce que** le système de navigation intégré, qui inclut le dispositif d'affichage, est fourni sous la forme d'un élément séparé pouvant être fixé sur l'instrument, lequel élément inclut une enveloppe stérile, en particulier un drapé stérile ou une coque stérile ou stérilisable.

11. Instrument selon l'une quelconque des revendications 1 à 10, **caractérisé en ce que** la partie de poignée ou de fixation et/ou la partie fonctionnelle et en particulier des composants de l'instrument fixés sur celle-ci sont formés comme des articles jetables stériles, en particulier comme des articles jetables emballés de manière stérile, notamment en matière plastique.
